# EUROPEAN PATENT SPECIFICATION

(11) **EP 0 544 056 B1**
(45) Date of publication and mention of the grant of the patent: **24.06.1998**
(21) Application number: 92111783.4
(22) Date of filing: 10.07.1992
(51) Int. Cl.: C07C 273/04

(54) **Process for urea production with differentiated yield reaction spaces**
Verfahren zur Herstellung von Harnstoff mit Reaktionsräumen unterschiedlicher Ausbeuten
Procédé pour la fabrication d'urée à espaces de réaction avec des rendements différentiés

(30) Priority: 14.11.1991 CH 3325/91
(43) Date of publication of application: 02.06.1993
(73) Proprietor: UREA CASALE S.A., CH-6900 Lugano (CH)
(72) Inventor: Pagani, Giorgio, I-20142 Milan (IT); Zardi, Umberto, Ch-6932 Breganzona (CH)
(74) Representative: Bottero, Claudio

(56) References cited:
- EP-A- 0 497 215
- DE-A- 1 643 092

## Description

This invention concerns a process for the industrial production of urea by reacting ammonia (NH₃) and carbon dioxide (CO₂) in a synthesis section consisting of many differentiated yield reaction spaces, in one of these spaces a majority part of the total conversion occurs by feeding it with highly pure reagents, and in the other of these spaces the remaining minority part of the conversion occurs by feeding it with less pure reagents that have been substantially recirculated by a recovery section.

A method for the synthesis of urea of the type set forth in the introduction is described in European patent application EP 0 479 103 corresponding to Swiss patent application No. 03216/90-1 deposited on October 3rd, 1990, by the same Applicant.

The plant for the carrying out of this process comprises: a first high-yield reactor fed with CO₂ and fresh NH₃ from the outside and with very pure recovery NH₃; a second reactor, parallel with the first, with a yield lower than the first and substantially fed with reagents from the recovery mixture; and a system or recovery section for the recovery of reaction mixtures obtained from said first and second reactors.

In European patent application EP 0 497 215, which is relevant only as to the novelty of the present invention, the Applicant has described an embodiment of the process according to said first patent application, which lends to a particularly efficient and advantageous result, because of the small investment and minimum consumption of energy it requires.

Characteristically, the majority synthesis reaction stage (A) with high-yield, between highly pure reagents, operating at a higher pressure (Pmax), for instance, above 300 bar abs and preferably around 400 bar abs, is followed by a flash stage F1 operating at pressures lower than approximately 200 bar abs, the gaseous effluent GF1 of the above mentioned flash stage F1 being fed to a minority synthesis reaction stage B with less pure reagents operating at a pressure lower than 200 bar abs, while the liquid effluent EL1 of the above mentioned flash stage, together with the effluent EB from minority reaction stage B operating in parallel to the majority reaction stage A, are fed to a recovery section RE consisting of two decomposition stages D-1 and D-2 operating in series: the first D-1 being lower than 100 bar abs preferably at 50 bar abs; the second D-2 working at a pressure lower than 50 bar abs preferably at 20 bar abs. Each decomposition stage consists of a decomposer D-1 and D-2 respectively (heat exchangers for the distillation of reagents not transformed into urea), whose gas effluents, consisting of NH₃+CO₂+H₂O, feed a condensation system with direct heat recovery from the process, where the partial condensation of said effluents is carried out and then completed in a fractionating column with a head condenser.

Additionally, a process for the synthesis of urea wherein NH₃ and CO₂ are reacted in a synthesis space implying A) a majority synthesis reaction between highly pure reagents and B) a minority synthesis reaction between less pure reagents and a decomposition stage between A) and B) has also been disclosed by DE-A-1 643 092.

In the uninterrupted research and experimentation in this important technical field, the Applicant had succeeded, not without surprise, to perfect an articulated and flexible process that will cope, with a particularly efficient and advantageous embodiment, with the most varied and frequent requirements by minimizing each time the investment costs and/or energy consumptions, and by maximizing the yields by adopting the most appropriate operating conditions according to the capacity of the plant.

These objectives are reached by a process according to claim 1 attached hereunder.

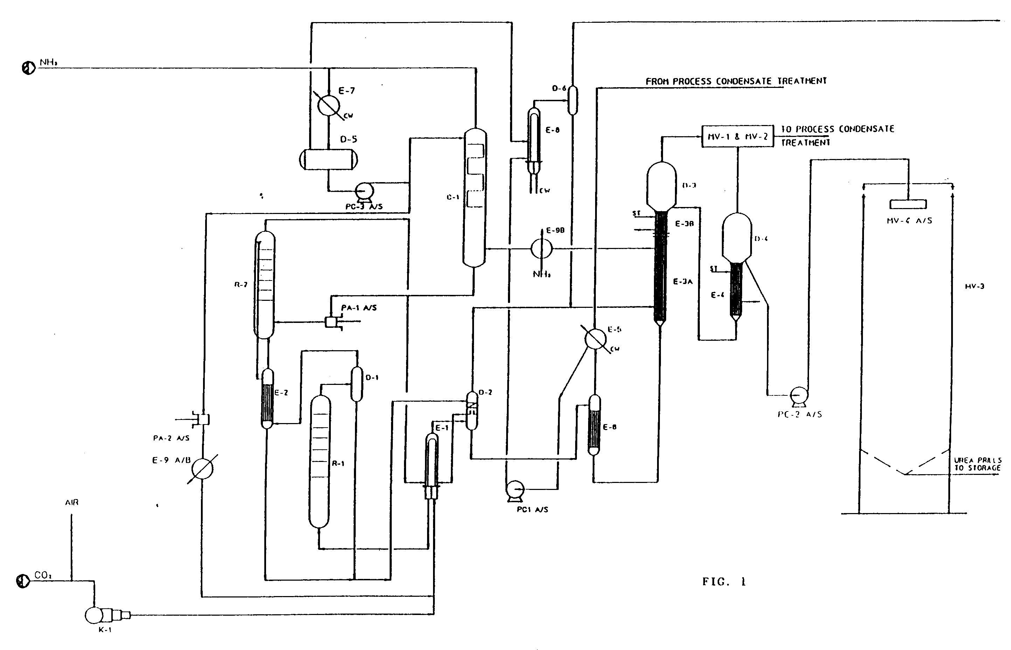

The various aspects and advantages of the invention will be better illustrated by the following description of one of the possible embodiments with heat removal, (preferred, but not limitative), shown in the attached drawing, in which Figure 1 is a process flow diagram.

### Differentiated Yield HEC (High Efficiency Combined) Process

This new process has the advantage of requiring low energy and inexpensive recycle units.

Moreover, the type of reactor for the reaction A) (a "once-through" type) has a high efficiency, great reliability, is substantially corrosion free, and requires low residence time i.e. small dimensions.

The plant for carrying out the process consists of the following sections:
a) synthesis section with two reactors (R-1, R-2) in parallel;
b) a medium pressure recycling section with one decomposition stage surmounted by an ammonia fractionating system to produce a purified urea solution, a carbamate solution and pure ammonia streams that are sent respectively to the secondary converter (R-2) (carbamate solution), and to the main converter (NH₃);
c) concentration section of the solution and for finishing.

### Heat Removal Process (Figure 1)

The main characteristic of the process is that the heat developed by the reaction 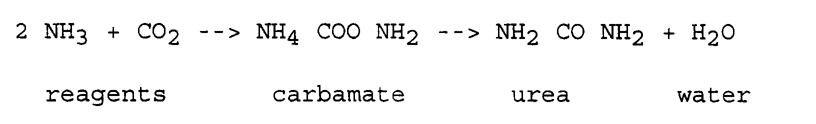 is utilized to bring the reagents to the reaction temperature.

The majority reaction A) is carried out in the following conditions:
- NH₃/CO₂ molar ratio: : 3.5 - 4
- H₂O/CO₂ molar ratio: : 0
- CO₂ conversion: : 80%
- NH₃ inlet temperature: : about 100°C
- CO₂ inlet temperature: : about 150°C
- Reaction temperature: : 195°C
- Pressure: : 240 bar abs
- Reactor conversion: : 75%
- Reactor lining: : AISI 316 L.U.G.

The urea solution from reactor R-1 undergoes flash from 240 to 150 bar in separator D-1.

The vapours produced in flash in D-1 (substantially ammonia) are sent to R-2, while the urea solution in D-1 is mixed with the solution from reactor R-2 and feeds the medium pressure decomposer E-1 downstream of D-2.

The operating conditions of the secondary reactor (R-2) are:
- NH₃/CO₂ molar ratio: : 4.5
- H₂O/CO₂ molar ratio: : 1.3
- CO₂ conversion yield: : 60%
- Pressure: : 150 bar abs
- Temperature: : 190°C

75% of the urea production is obtained in R-1, whereas the remaining 25% is obtained in R-2.

The weighed average efficiency of the two reactors is close to 71.5%, that is very high in comparison to the efficiency of very recent processes.

Characteristically, the CO₂ and fresh ammonia are fed by a medium pressure decomposer-prereactor E-1 tube side, in which ammonium carbamate and urea are formed.

E-1 is part of the majority reaction stage A2) with R-1. A part of the reaction heat is removed from the urea solution that circulates outside said tubes.

Advantageously, this heat has a high thermal level (about 170°C) that is exploited for the distillation of the same urea solution in decomposer E-1 operating at 18 bar that can be of the falling-film type or up-flow type ("process-to-process heat exchange").

The carbamate/urea solution in the pre-reactor E-1 is sent to reactor R-1, in which the majority part of the carbamate is dehydrated to urea. Characteristically, the control of the heat developed in E-1 is carried out regulating the temperature of liquid ammonia through E-9, while the temperature of R-1 is maintained by-passing, if necessary, the CO₂ to E-1.

The auxiliary reactor R-2 is fed with flash vapours and with the recycled carbamate solution, as well as with the vapours coming from the high pressure decomposer E-2, preferably of falling-film type, wherein the heat is given by means of medium pressure steam with the aim of performing the thermal balance of reactor R-2 and to distill the solution coming from R-2.

The urea solution leaving D-2 is then distilled in decomposer E-6 operating at 3.5 bar. The urea solution then passes in the vacuum section where it is concentrated up to 96% w in the first evaporator E-3 that operates at 0.35 bar, and then up to 99.7% w in the second evaporator E-4 operating at 0.05 bar.

The vapours obtained in D-2 are partially condensed in the first part of the first evaporator E-3A, in which a part of the process heat is recovered (with a double-effect system), and are sent to the fractionating column C-1. In the latter pratically all the CO₂ and H₂O vapours are condensed as carbamate solution and sent to reactor R-2.

Pure NH₃ at the head of column C-1 is condensed in E-7 and the liquid ammonia obtained is used, together with the fresh NH₃ feed, as reflux for column C-1 and feeds pre-reactor E-1. The quantity of NH₃ reflux is determined by the thermal balance of column C-1.

### Consumptions

The specific consumptions, referring to 1000 kg of urea, are:
- Liquid NH₃ at 32°C, 18 bar (kg): : 568
- CO₂ (kg): : 734
- Steam at 25 bar (kg): : 400*
- Electric power (kWh): : 115

* excluding water formation treatment.

### Example

Described hereunder, with a demonstrative aim, but not limitative, is the application of the invention to modernize existing Weatherly type plants.

These plants, developed in the U.S.A. in the years 1960 - 1970, are characterized by the fact that the synthesis reactor is fed with pure reagents (NH₃ and CO₂) without recycle water, which has enabled these plants to obtain very high conversion yields of CO₂ in urea (75%).

The operating data of these Weatherly reactors are:
- - NH₃/CO₂ molar ratio: : 4
- - H₂O/CO₂ molar ratio: : 0
- - yield: : 75%
- - pressure: : 260 bar abs
- - temperature: : 195°C

The process of this invention also provides a simple, inexpensive, efficient and convenient extension of its European application EP 0 479 103 to the existing urea synthesis plant of the "once through" type, mainly Weatherly processes. Particularly, the Weatherly synthesis section of these plants represents the majority portion described herein.

In fact, it has been found that it is surprisingly possible to revamp, in a simple and safe manner, using this invention's processes as described and claimed, existing urea production plants of the "once through" type.

These "once through" plants present the following main disadvantages :
- partial utilization of the CO₂ fed to the reactor, connected to the yield of the reactor itself (in the Weatherly reactor, with a yield equal to 75%, only 75% of the fed CO₂ is converted into urea), whereas in total recycle plants the utilization CO₂ yield is equal to approximately 100%;
- very high energy consumptions in the case of residual CO₂ recovery through the use of an absorbing solution as the MEA type;
- very little operating flexibility, the urea plant's run being bound to the utilization of gaseous effluents (vapours rich in NH₃) in other plants.

Among the main advantages of the process according to the invention, the following are mentioned:
1) High urea yield in the synthesis section with consequent recycle sections downstream simple and inexpensive.
2) Elimination of all critical equipment subject to corrosion, used in modern stripping processes, such as strippers, high pressure carbamate condensers, scrubbers, etc., resulting in a longer life of the plant.
3) Very low carbamate recycle to the low pressure converter (with respect to conventional high pressure total recycle processes) and very small carbamate pumps.
4) Very low utility and energy consumptions.
5) Absence of recovery steam generation in the plant with consequently minor heat transfer surface requirements, and absence of recovery steam to be used elsewhere.
6) Reduced investment costs.

## Claims

1. A process of producing urea in a plant including at least one synthesis space (SS) for reacting ammonia and carbon dioxide at high temperature and pressure and a recovery section for recovering unreacted reagents, comprising the steps of:
(a) reacting highly pure ammonia and carbon dioxide in a first reaction stage (E-1, R-1) at a predermined pressure and temperature sufficient to carry out the reaction;
(b) flash separating a product stream from the first reaction stage (E-1, R-1) into ammonia-containing vapours and a first liquid effluent including urea;
(c) reacting the ammonia-containing vapours thus obtained and a carbamate solution recycled from the recovery section in a second reaction stage (R-2) at a pressure less than 200 kg/cm² and at a temperature sufficient to carry out the reaction so as to obtain a second liquid effluent including urea;
(d) feeding the liquid effluents including urea from said first (E-1, R-1) and second (R-2) reaction stages to the recovery section, decomposing the liquid effluents in the recovery section and withdrawing a purified urea solution therefrom,
wherein the highly pure ammonia and carbon dioxide are reacted in said first reaction stage (E-1, R-1) with partial removal of the reaction heat at a pressure less than 300 kg/cm², at a temperature not higher than 200°C and at an ammonia/carbon dioxide ratio less than 4.

2. A process according to claim 1, wherein the first reaction stage (E-1, R-1) operates at a pressure of 240 bar abs.

3. A process according to claim 1, wherein the product stream from the first reaction stage (E-1, R-1) is flash-separated at a pressure equal to the synthesis pressure in the second reaction stage (R-2).

4. A process according to claim 3, wherein said flash separation is carried out at a pressure of 150 bar.

5. A process according to claim 1, wherein 75% of the urea is produced in the first reaction stage (E-1, R-1) and 25% of the urea is produced in the second reaction stage (R-2).

6. A process according to claim 1, wherein the reaction between highly pure ammonia and carbon dioxide in said first reaction stage (E-1, R-1) is carried out in a decomposer-prereactor (E-1) and in a synthesis reactor (R-1) in series, the reaction heat being partially removed in said decomposer-prereactor (E-1).

7. A process according to claim 6, wherein the liquid effluents including urea from said first (E-1, R-1) and second (R-2) reaction stages are distilled in said decomposer-prereactor (E-1) by means of the reaction heat removed therein from the urea solution that circulates outside the tubes of said decomposer-prereactor (E-1).

8. A process according to claim 6, wherein the removal of the reaction heat in said decomposer-prereactor (E-1) is controlled by regulating the temperature of the ammonia stream recycled from the recovery section.

9. A process according to claim 6, wherein the temperature of said first reaction stage (E-1, R-1) is maintained by by-passing the highly pure carbon dioxide to said decomposer-prereactor (E-1).

10. A process according to claim 1, further comprising the step of recycling to the first reaction stage (E-1, R-1) an ammonia stream obtained by decomposing in the recovery section the liquid effluents from said first (E-1, R-1) and second (R-2) reaction stages.

11. A process according to claim 1, further comprising the step of distilling the liquid effluent from the second reaction stage (R-2) in a high pressure decomposer (E-2).

12. A process according to claim 11, wherein said distillation step is carried out by countercurrently contacting in the high pressure decomposer (E-2) the liquid effluent including urea from the second reaction stage (R-2) and the flash-separated ammonia-containing vapours from the first reaction stage (E-1, R-1).

## Patentansprüche

1. Verfahren zum Herstellen von Harnstoff in einer Anlage, welche zumindest einen Syntheseraum (SS) zum Durchführen einer Reaktion von Ammoniak und Kohlendioxid unter hoher Temperatur und Druck und einen Rückgewinnungsabschnitt zum Rückgewinnen von nicht reagierten Reagenzien enthält, welches die folgenden Schritte aufweist:
(a) Zur-Reaktion-Bringen von hochreinem Ammoniak und Kohlendioxid in einer ersten Reaktionsstufe (E-1, R-1) bei einem vorbestimmten Druck und einer vorbestimmten Temperatur, ausreichend, um die Reaktion durchzuführen,
(b) Flash-Auftrennung eines Produktstromes von der ersten Reaktionsstufe (E-1, R-1) in ammoniakhaltige Dämpfe und einen ersten flüssigen Ausfluß, welcher Harnstoff enthält,
(c) Zur-Reaktion-Bringen der so gewonnenen ammoniakhaltigen Dämpfe und einer Carbamatlösung, welche von dem Rückgewinnungsabschnitt rückgeführt wird, in einer zweiten Reaktionsstufe (R-2) bei einem Druck von weniger als 200 kg/cm² und einer Temperatur, welche ausreicht, um die Reaktion durchzuführen, so daß man einen zweiten flüssigen Ausfluß erhält, welcher Harnstoff enthält,
(d) Zuführen der harnstoffhaltigen flüssigen Ausflüsse von der ersten (E-1, R-1) und zweiten (R-2) Reaktionsstufe zu dem Rückgewinnungsabschnitt, Zersetzen der flüssigen Ausflüsse in dem Rückgewinnungsabschnitt und Entnehmen einer gereinigten Harnstofflösung hieraus,
wobei der hochreine Ammoniak und Kohlendioxid in der ersten Reaktionsstufe (E-1, R-1) unter einer teilweisen Abführung der Reaktionswärme bei einem Druck von weniger als 300 kg/cm², einer Temperatur von nicht mehr als 200 °C und einem Ammoniak/Kohlendioxid-Verhältnis von weniger als 4 zur Reaktion gebracht werden.

2. Verfahren nach Anspruch 1, bei dem die erste Reaktionsstufe (E-1, R-1) bei einem Absolutdruck von 240 bar arbeitet.

3. Verfahren nach Anspruch 1, bei dem der Produktstrom von der ersten Reaktionsstufe (E-1, R-1) bei einem Druck gleich dem Synthesedruck in der zweiten Reaktionsstufe (R-2) flash-aufgetrennt wird.

4. Verfahren nach Anspruch 3, bei dem die Flash-Auftrennung bei einem Druck von 150 bar ausgeführt wird.

5. Verfahren nach Anspruch 1, bei dem 75 % des Harnstoffs in der ersten Reaktionsstufe (E-1, R-1) und 25 % des Harnstoffs in der zweiten Reaktionsstufe (R-2) produziert werden.

6. Verfahren nach Anspruch 1, bei dem die Reaktion zwischen hochreinem Ammoniak und Kohlendioxid in der ersten Reaktionsstufe (E-1, R-1) in einem Zersetzer-Vorreaktor (E-1) und in einem Synthesereaktor (R-1) in Reihe durchgeführt wird, wobei die Reaktionswärme in dem Zersetzer-Vorreaktor (E-1) teilweise entzogen wird.

7. Verfahren nach Anspruch 6, bei dem die harnstoffhaltigen flüssigen Ausflüsse von der ersten (E-1, R-1) und zweiten (R-2) Reaktionsstufe in dem Zersetzer-Vorreaktor (E-1) mit Hilfe der Reaktionswärme destilliert werden, welche darin der Harnstofflösung entzogen wird, welche außerhalb der Rohre des Zersetzer-Vorreaktors (E-1) zirkuliert.

8. Verfahren nach Anspruch 6, bei dem das Entziehen der Reaktionswärme in dem Zersetzer-Vorreaktor (E-1) dadurch kontrolliert wird, daß die Temperatur des Ammoniakstroms reguliert wird, der von dem Rückgewinnungsabschnitt rückgeführt wird.

9. Verfahren nach Anspruch 6, bei dem die Temperatur der ersten Reaktionsstufe (E-1, R-1) aufrechterhalten wird, indem das hochreine Kohlendioxid im Bypass zu dem Zersetzer-Vorreaktor (E-1) geleitet wird.

10. Verfahren nach Anspruch 1, welches weiterhin den Schritt des Rückführens eines Ammoniakstroms zu der ersten Reaktionsstufe (E-1, R-1) enthält, den man durch Zersetzen der flüssigen Ausflüsse von der ersten (E-1, R-1) und der zweiten (R-2) Reaktionsstufe in dem Rückgewinnungsabschnitt gewinnt.

11. Verfahren nach Anspruch 1, welches weiterhin den Schritt des Destillierens des flüssigen Ausflusses von der zweiten Reaktionsstufe (R-2) in einem Hochdruckzersetzer (E-2) aufweist.

12. Verfahren nach Anspruch 11, bei dem der Destillationsschritt durchgeführt wird, indem in dem Hochdruckzersetzer (E-2) der harnstoffhaltige flüssige Ausfluß von der zweiten Reaktionsstufe (R-2) und die flash-abgetrennten harnstoffhaltigen Dämpfe von der ersten Reaktionsstufe (E-1, R-1) im Gegenstrom miteinander in Kontakt gebracht werden.

## Revendications

1. Procédé de production durée dans une usine incluant au moins un espace de synthèse (SS) pour faire réagir de l'ammoniac et du dioxyde de carbone, à température et à pression élevées et une section de récupération pour récupérer des réactifs n'ayant pas réagi, comprenant les étapes consistant à :
(a) faire réagir de l'ammoniac et du dioxyde de carbone extrêmement purs durant une première étape réactionnelle (E-1, R-1), à une pression et à une température prédéterminées suffisantes pour effectuer la réaction ;
(b) effectuer une séparation flash d'un courant de produit provenant de la première étape réactionnelle (E-1, R-1) en des vapeurs contenant de l'ammoniac et un premier effluent liquide incluant de l'urée ;
(c) faire réagir les vapeurs contenant de l'ammoniac ainsi obtenues et une solution de carbamate recyclée provenant de la section de récupération durant une seconde étape réactionnelle (R-2), à une pression inférieure à 200 kg/cm² et à une température suffisante pour effectuer la réaction, de façon à obtenir un second effluent liquide incluant de l'urée ;
(d) introduire les effluents liquides incluant de l'urée provenant desdites première (E-1, R-1) et seconde (R-2) étapes réactionnelles dans la section de récupération, décomposer les effluents liquides dans la section de récupération et en soutirer une solution d'urée purifiée,
dans lequel on fait réagir l'ammoniac et le dioxyde de carbone extrêmement purs durant ladite première étape réactionnelle (E-1, R-1), avec élimination partielle de la chaleur de la réaction, à une pression inférieure à 300 kg/cm², à une température non supérieure à 200°C et selon un rapport ammoniac/dioxyde de carbone inférieur à 4.

2. Procédé selon la revendication 1, dans lequel la première étape réactionnelle (E-1, R-1) fonctionne à une pression de 240 bars absolus.

3. Procédé selon la revendication 1, dans lequel le courant de produit provenant de la première étape réactionnelle (E-1, R-1) subit une séparation flash à une pression égale à la pression de synthèse dans la seconde étape réactionnelle (R-2).

4. Procédé selon la revendication 3, dans lequel ladite séparation flash est effectuée à une pression de 150 bars.

5. Procédé selon la revendication 1, dans lequel 75% de l'urée est produite durant la première étape réactionnelle (E-1, R-1) et 25% de l'urée est produite durant la seconde étape réactionnelle (R-2).

6. Procédé selon la revendication 1, dans lequel la réaction entre l'ammoniac et le dioxyde de carbone extrêmement purs dans ladite première étape réactionnelle (E-1, R-1) est effectuée dans un appareil de fractionnement - pré-réacteur (E-1) et dans un réacteur de synthèse (R-1) en série, la chaleur de la réaction étant partiellement éliminée dans ledit appareil de fractionnement - pré-réacteur (E-1).

7. Procédé selon la revendication 6, dans lequel les effluents liquides incluant de l'urée provenant desdites première (E-1, R-1) et seconde (R-2) étapes réactionnelles sont distillés dans ledit appareil de fractionnement - pré-réacteur (E-1) grâce à la chaleur de la réaction éliminée de celui-ci à partir de la solution d'urée qui circule à l'extérieur des tubes dudit appareil de fractionnement - pré-réacteur (E-1).

8. Procédé selon la revendication 6, dans lequel l'élimination de la chaleur de la réaction dans ledit appareil de fractionnement - pré-réacteur (E-1) est contrôlée en régulant la température du courant d'ammoniac recyclé depuis la section de récupération.

9. Procédé selon la revendication 6, dans lequel la température de ladite première étape réactionnelle (E-1, R-1) est maintenue en faisant repasser le dioxyde de carbone extrêmement pur vers ledit appareil de fractionnement - pré-réacteur (E-1).

10. Procédé selon la revendication 1, comprenant en outre l'étape de recyclage vers la première étape réactionnelle (E-1, R-1) d'un courant d'ammoniac obtenu en décomposant, dans la section de récupération, les effluents liquides provenant desdites première (E-1, R-1) et seconde (R-2) étapes réactionnelles

11. Procédé selon la revendication 1, comprenant en outre l'étape de distillation de l'effluent liquide provenant de la seconde étape réactionnelle (R-2) dans un appareil de fractionnement à haute pression (E-2).

12. Procédé selon la revendication 11, dans lequel ladite étape de distillation est effectuée en mettant en contact à contre-courant, dans l'appareil de fractionnement à haute pression (E-2), l'effluent liquide incluant de l'urée provenant de la seconde étape réactionnelle (R-2) et les vapeurs contenant de l'ammoniac séparées par séparation flash provenant de la première étape réactionnelle (E-1, R-1).
